# EUROPEAN PATENT APPLICATION

(11) **EP 4 582 009 A2**
(43) Date of publication of application: **09.07.2025**
(21) Application number: 24221873.3
(22) Date of filing: 19.12.2024
(51) Int. Cl.: A61B 1/00, A61B 1/31

(54) **MEDICAL DEVICE ACCESSORY**

(30) Priority: 02.01.2024 GB 202400012
(71) Applicant: KEYMED (Medical & Industrial Equipment) Limited, Southend-on-Sea Essex SS2 5QH (GB)
(72) Inventor: Bayer, Andreas, SOUTHEND-ON-SEA, SS2 5QH (GB); Jowitt, Jordan, SOUTHEND-ON-SEA, SS2 5QH (GB)
(74) Representative: Boult Wade Tennant LLP

(57) **Abstract**

A cover for fitting over a distal end of a shaft of a medical device is provided. The cover comprises: a tubular body with a first end, a second end, inner and outer circumferential surfaces, an internal diameter and at least one projecting element extending outwardly; and an axial slot in the tubular body extending at least partially between the first end and the second end, wherein the tubular body is movable between a first position in which the slot has a first width and the tubular body has a first internal diameter, and a second position in which the width of the slot and the internal diameter of the tubular body are increased, wherein the tubular body is resiliently biased from the second position towards the first position.

## Description

### FIELD

The present disclosure relates to an accessory for use with a medical device, such as an endoscope.

### BACKGROUND

US 2019/0183328 A1 describes a cover for the shaft of a medical device, in particular for fitting over the distal tip of an endoscope. The cover is a tubular member with proximal and distal ends and inner and outer circumferential surfaces. Axially extending ribs may be provided on the inner and outer circumferential surfaces. A plurality of projecting elements is provided on the outer surface of the cover which in use extend radially outwardly from the cover. These can pull back or flatten folds of body tissue to facilitate the inspection of a body cavity or carrying out other medical procedures with the medical device.

This type of cover is formed of a plastic material and forms a friction fit on the shaft of the medical device. However, as the external dimensions of a shaft of a medical device may vary slightly, for example with different models, there is a need to provide a cover which can adapt to shafts of different sizes while maintaining a secure fit on the shaft.

US 2005/0234297 A1 discloses, according its abstract, devices and methods for detachably engaging an insertion section of an endoscope and selectively articulating an endoscopic surgical access channel.

US 2022/0160213 A1 discloses, according to its abstract, an endoscopic device which includes at least an endoscope and one or more catheters, and a means for securing the endoscope to the catheter at a distal end of the endoscopic device.

US 2014/0296629 A1 discloses, according to its abstract, systems, methods, and devices that include a releasable mount device that may be utilized to couple an operative element, such as an ablation device, with a therapeutic or diagnostic device, such as an endoscope.

US 2023/0137851 A1 discloses, according to its abstract, an endoscopic retraction assist device which includes a body comprising opposite proximal and distal end portions and a central portion between the proximal and distal end portions, and a device channel defined in the body.

US 6 569 085 B2 discloses, according to its abstract, methods and apparatus for delivering a medical instrument over the exterior of an endoscope while the endoscope is installed in the patient's body.

### SUMMARY

A cover for fitting over the distal end of a shaft of a medical device is provided. The cover comprises: a tubular body with a first end, a second end, inner and outer circumferential surfaces, an internal diameter and at least one projecting element extending outwardly; and an axial slot in the tubular body extending at least partially between the first end and the second end, wherein the tubular body is movable between a first position in which the slot has a first width and the tubular body has a first internal diameter, and a second position in which the width of the slot and the internal diameter of the tubular body are increased, wherein the tubular body is resiliently biased from the second position towards (or into) the first position. Such a cover can be readily fitted onto various medical device shafts.

The axial slot may extend a full length of the tubular body between the first end and the second end. This can allow for a large amount of variation in the respective sizes between the two positions.

The axial slot may be a partial axial slot. That is, the slot may not axially extend an entire axial length of the tubular body.

The tubular body may comprise a hinge section aligned circumferentially with the partial axial slot. This allows a remaining portion of the tubular body to hinge between the two positions.

The cover may comprise a plurality of axial slots. This can include the axial slot noted above. A plurality of axial slots can allow for more variation in the respective sizes between the two positions.

The axial slots may be arranged with rotational symmetry about the tubular body. This can allow for equal deviation between parts of the tubular body separated by the axial slots.

The tubular body may further comprise at least one biasing member. The biasing member can specifically be a spring member (or spring element) in any of the examples discussed herein. The biasing member can bias the tubular body towards the first position.

The biasing member may comprise a resilient spring plate located between the inner and outer circumferential surfaces of the tubular body. Such a plate can effectively deliver the required bias.

The spring plate may be perforated. This can reduce weight of the spring plate.

The spring plate may comprise a plurality of circumferentially extending bands joined by at least one axially extending connecting bar. Again, this can help reduce the weight of the spring plate.

The biasing member may comprise a plurality of separate circumferentially extending bands. This is a lightweight and effective way to bias the tubular body.

The biasing member may be formed of metal. This can be advantageous as a metal will typically not behave viscoelastically (i.e. in a viscoelastic manner) - particularly when compared to a plastic which may form a coating of the tubular body. This helps ensure that the biasing member retains its tension over time. For example, the cover may be pretensioned in its packaging.

The biasing member may be embedded within a flexible coating material. This can protect the biasing member, and ensure that it does not contact a patient when the cover is used *in vitro.*

The biasing member may be moulded in one piece with a flexible coating material. This is an effective way to manufacture the cover.

The axial slot may be non-linear. This can help ensure the cover is aligned when installed.

The axial slot may be angled with respect to a radial direction of the tubular body. This means that there is not a radial gap through the cover.

The axial slot may increase in width towards the second end of the tubular body. This can allow for effective manipulation between the two positions, while maintaining a suitable grip to the shaft.

The cover may further comprise a flexible bridging element spanning at least a portion of the axial slot. This bridging element may act as the biasing member discussed herein.

The flexible bridging element may be axially discontinuous. This can reduce the weight of the cover.

A holder for the cover as discussed herein is provided. The holder comprises: a base and a side wall together defining an enclosure for receiving the cover; and a blocking member projecting into the enclosure and configured to fit within the axial slot in the tubular body when the tubular body is in the second position, to maintain the tubular body in the second position. This holder can allow for easy installation of the cover onto a shaft.

The blocking member may comprise a main body with a nose portion, wherein the nose portion is narrower than the main body and is configured to fit into the axial slot in the tubular body. This holds the axial slot open, allowing the cover to be fitted easily.

The holder may further comprise at least one locating member projecting into the enclosure opposite to the blocking member. The locating member helps properly position the shaft and the cover.

The blocking member may extend upwardly from the base to a first height, the or each locating member may extend upwardly from the base to a second height, and the first height may be greater than the second height. This allows a tilting movement to remove the nose portion from the slot, thereby facilitating installation of the cover to the shaft.

The nose portion may comprise parallel side walls.

The nose portion may comprise diverging side walls. This can help retain the cover to the holder.

The nose portion may comprise a concave end face. This aids in receiving the shaft in the cover.

An assembly is provided comprising the cover as discussed herein fitted into a holder as discussed herein. This assembly facilitates easy installation of the cover to the shaft.

A method for fitting the cover as discussed herein to a distal end of a shaft of a medical device is provided, wherein the cover is fitted into the holder as discussed herein such that the blocking member locates in the axial slot of the tubular body to maintain the cover in the second position, and the method comprises: inserting the distal end of a shaft of a medical device into the tubular body; tilting the shaft to remove the blocking member from the axial slot and to permit the tubular body to return to the first position and to grip the shaft; and removing the shaft and cover from the holder. As such, the cover is easily installed to the shaft.

### BRIEF DESCRIPTION OF THE DRAWINGS

The present disclosure refers, by way of example only, to the accompanying drawings in which:
Figure 1 is a perspective view of one embodiment of accessory, in a first, closed position;
Figure 2 is a side view of the accessory of Figure 1;
Figure 3 is a plan view of the accessory of Figure 1;
Figure 4 shows perspective, side and plan views of a spring member;
Figure 5 shows a perspective view and a plan view of the accessory of Figures 1 to 4 in a second, open position;
Figure 6a shows a perspective view from above and a plan view of one embodiment of a holder for the accessory of Figure 1;
Figure 6b shows a plan view of a second embodiment of a holder;
Figures 7 shows a perspective view from below and a side view of the holder of Figure 6a;
Figure 8 shows a plan view and a perspective view from above of the holder of Figures 6a and 7, with the accessory fitted therein;
Figures 9a to d illustrate fitting a shaft into an accessory fitted in a holder;
Figures 10a to 10i illustrate alternative forms of spring member;
Figure 11a shows a perspective view of an accessory, in a first, closed position;
Figure 11b shows a perspective view of the accessory of Figure 11a in a second, open position;
Figures 12a to 12c show perspective, top, and close-up perspective views respectively of an accessory in a first, closed position;
Figure 13 shows a perspective view of an accessory in a first, closed position;
Figures 14a to 14c show perspective, top, and close-up perspective views respectively of an accessory in a first, closed position;
Figures 15a to 15c show perspective, top, and close-up perspective views respectively of an accessory in a first, closed position;
Figure 16 shows a perspective view of an accessory in a first, closed position;
Figure 17 shows a perspective view of an accessory in a first, closed position;
Figures 18a to 18b show perspective and close-up side views respectively of an accessory in a first, closed position;
Figures 19a to 19b show perspective and close-up front views respectively of an accessory in a first, closed position.
Figures 20a to 20c show perspective, top, and front views respectively of an accessory in a first, closed position
Figures 21a to 21b show first and second perspective views of an accessory in a first, closed position.

### DETAILED DESCRIPTION

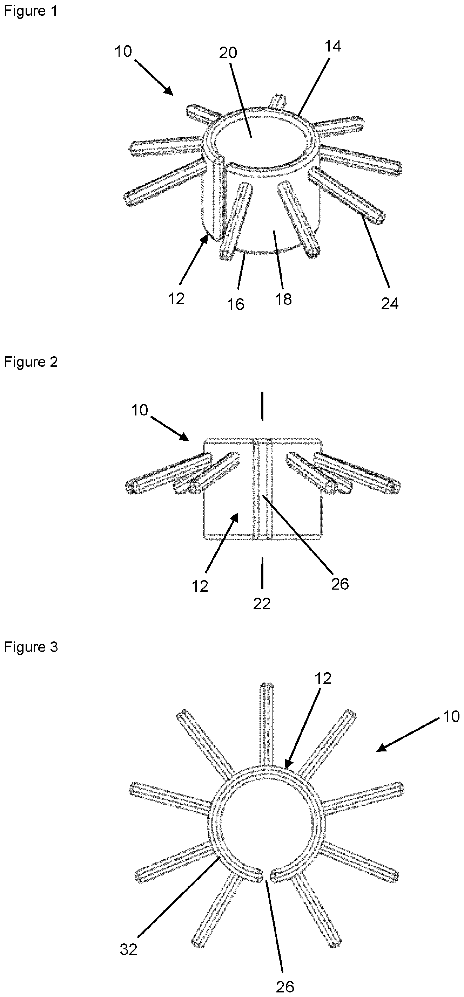

As shown in Figures 1 to 3, an accessory 10 for a medical device comprises a cover for fitting, in particular, over the distal end of a shaft 70 of the medical device. The distal end being the end of the shaft 70 of the medical device which is first inserted into a patient in use.

In this disclosure the term "medical device" may refer to an endoscope, but this term is also intended to refer to any device suitable for insertion into a biological cavity or lumen in order to carry out visualisation thereof and/or treatment. Accordingly, the term "medical device" is intended to encompass any or all of endoscopes, gastroscopes, colonoscopes, enteroscopes, sigmoidoscopes, panendoscopes, but this list is non-exhaustive. Endoscopy involves inspecting the inside of a body lumen or cavity and includes procedures known as arthroscopy, cystoscopy, gastroscopy, uteroscopy and colonoscopy. Enteroscopy involves examination of the small intestine including the duodenum, jejunum and ileum. Such devices are elongate flexible probes which may be inserted into the body directly or via a cannula or other guide device. Medical devices can also include rigid surgical endoscopes or endotherapy devices such as biopsy forceps and polypectomy snares. The cover of the present disclosure may be used in conjunction with all of the aforementioned types of medical device. The medical device may specifically be a medical scoping device.

The cover comprises a tubular body 12 with first and second ends 14, 16, an outer circumferential surface 18, an inner circumferential surface 20 and a longitudinal axis 22. The accessory 10 also includes one or more projecting elements 24 projecting radially outwardly, as described further below. In other words, the tubular body 12 is a (partial) hollow cylinder. Although the body 12 is referred to as a tubular body 12, as shown in Figures 1 to 3, it does not form a full cylindrical surface. In other words, the accessory 10 and/or the tubular body 12 are discontinuous.

The tubular body 12 may extend through at least 270° in plan view such as shown in Figure 3. Preferably, this may be at least 300° or even at least 330°.

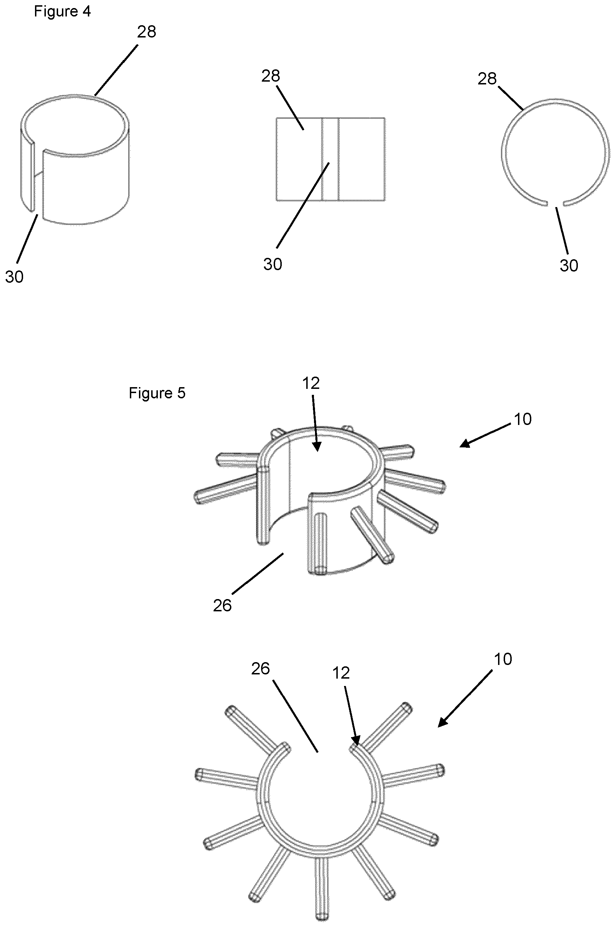

The tubular body 12 is open at its first and second ends 14, 16. The tubular body 12 also comprises a slot 26 extending axially between the first end 14 and the second end 16. The slot 26 may be axially aligned in that it extends only in the axial direction. Alternatively, the slot 26 may simply have an axial extend and may also extend in a circumferential direction. The tubular body 12 is flexible and resilient such that it can be bent or deformed to open up the axial slot 26. This increases the width of the slot 26 and to increase the internal diameter of the tubular body 12, as shown in Figure 5.

As explained in more detail below, a bridging element 27 may be provided to span at least a portion of the slot 26. However, the resiliently deformably tubular body 12 is still discontinuous with the slot 26.

The tubular body 12 preferably comprises a biasing member 28 which, when at rest and in an un-tensioned state, retains the tubular body 12 in a first, closed position as in Figures 1 to 3, in which the tubular body 12 has a first internal diameter and the axial slot 26 has a first width. The biasing member 28 may specifically be a spring member 28 (also identified as a spring element 28). However, any reference to a spring member 28 in the present description is equally applicable to a general biasing member 28. The first width may be very small, or substantially zero with the sides of the slot 26 contacting each other. For example, in plan view the axial slot 26 may extend over no more than 90°, preferably no more than 60°, more preferably no more than 30°. The first internal diameter is sufficient to allow the tubular body 12 to encircle and grip a shaft 70.

The tubular body 12 may be deformed into a second, open position by bending to open up the axial slot 26 in a circumferential direction to a second width, larger than the first width, as shown in Figure 5. This places the spring member 28 in tension and the tubular body 12 in a second position in which the tubular body 12 has a second internal diameter, larger than the first internal diameter. The spring member 28 biases the tubular body 12 towards the first, closed position. Once a deflecting force acting to open up the axial slot 26 is removed, the accessory 10 will return to its first, closed position, in which the tubular body 12 returns to its first internal diameter and the axial slot 26 returns to its first width.

In the embodiment shown, the spring member 28 comprises a spring plate. The plate may be a rectangle of resilient material, which is bent round into a tubular shape, leaving an axially extending opening 30 between its free ends, as shown in Figure 4, so that in plan view it is generally C-shaped. Preferably, the spring member 28 is formed of metal, such as spring steel, which maintains its tension over time (if only loaded within its elastic range). However, other resilient materials may be used which have a high yield strength so that the spring member 28 will return to the initial tubular shape when a deflecting force acting to open up the axial opening 30 is removed.

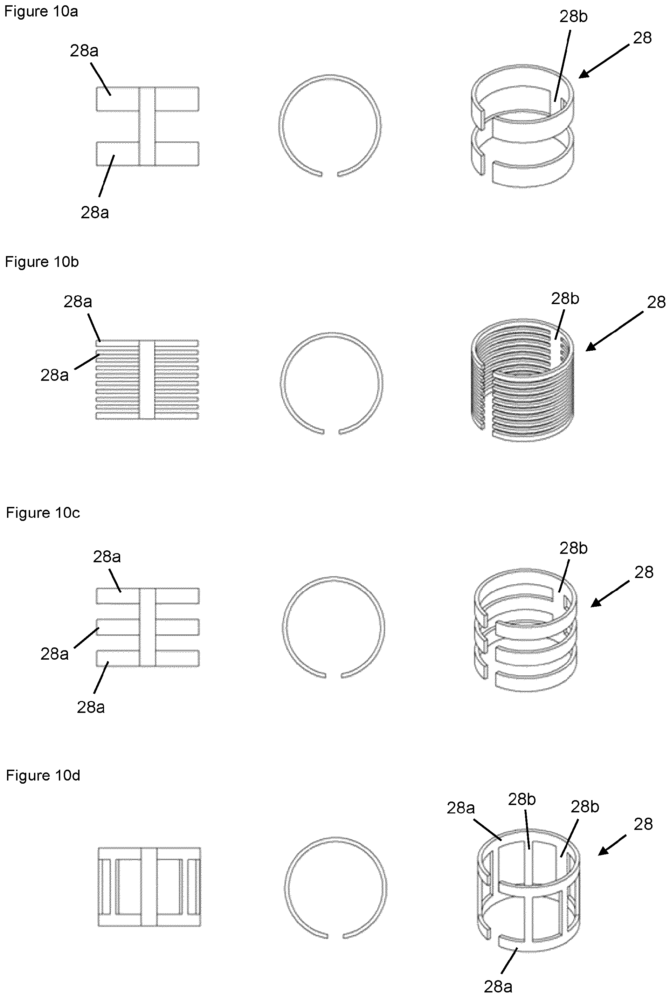

The spring member 28 may be configured in different ways if desired. For example, while Figure 4 shows a solid plate, the spring member may be formed of a perforated sheet with one or more openings, in order to reduce weight. Various examples are shown in Figures 10a to 10h, in which the spring member 28 comprises a number of circumferentially extending bands 28a, with gaps between them, joined by one or more axially extending connecting bars 28b.

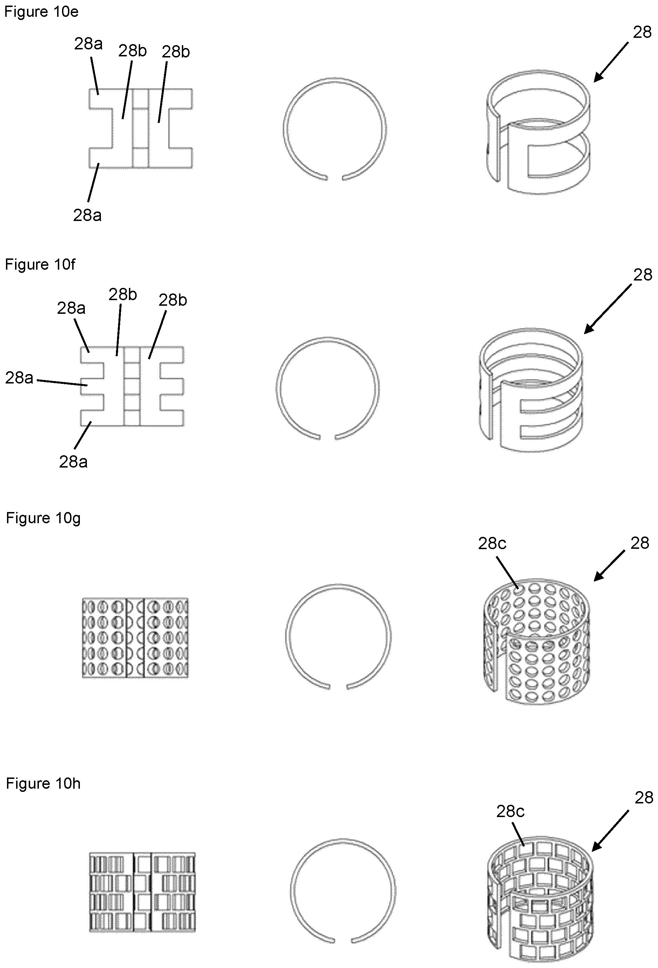

A connecting bar 28b may be formed at a location opposite to the axial slot 26 as in Figures 10a to 10c. A pair of connecting bars 28b may be provided at the ends of the spring plate 28, defining the opening 30 between them, as in the examples of Figures 10e and 10f. Multiple connecting bars 28b may be provided, spaced around the circumference as in Figure 10d.

In Figures 10g and 10h, the spring plate 28 is provided with an array of perforations, such a circular or square/rectangular openings 28c.

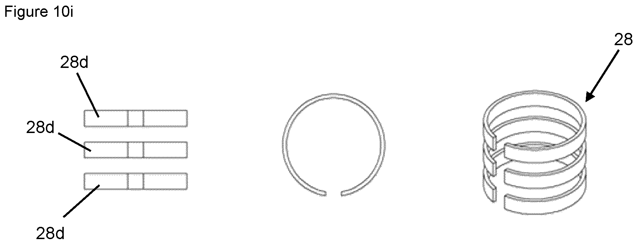

Alternatively, the spring member 28 may comprise a series of separate C-shaped bands 28d, stacked one above the other in an axial direction, as in Figure 10i.

The spring member 28 may be completely encased within a coating material 32, such as a plastic or other polymer. This coating material my be a softer, flexible coating material 32, such as a plastic or other polymer. In other words, the spring member 28 may be embedded within the coating material 32.

The spring member 28 and the coating material 32 may be moulded together in one piece. This softer coating material 32 forms the exterior surface of the tubular body 12, including the outer and inner circumferential surfaces 18, 20. The softer coating material 32 protects the shaft 70 and may enhance the grip of the tubular body 12 on the shaft 70. The coating material 32 may also be textured or shaped, for example with ribs on the outer and/or inner circumferential surfaces 18, 20.

The annular projecting element 24 may be located at or near to the first end 14, and may comprise a plurality of arms projecting radially outwardly from the tubular body 12. The arms may be identical to each other and equally spaced around the tubular body 12, but other configurations are also possible. Connecting webs (not shown) may join adjacent arms to one another. Alternatively, the projecting element 24 may comprise an annular collar extending around the circumference of the tubular body 12, except for across the axial slot 26.

In all embodiments, the projecting element 24 may be made of a resilient polymer material, such as a silicone. The projecting element 24 therefore has some stiffness and is self-supporting so that it maintains its shape, while allowing some flexing as it contacts body tissue when in use.

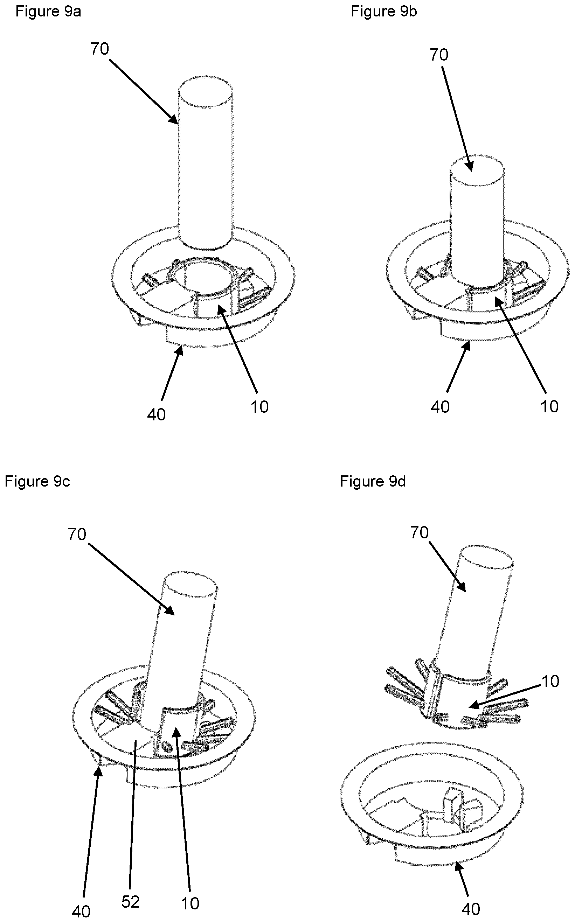

In use, the accessory 10 is fitted over the distal end of a shaft 70 of a medical device such as an endoscope, as shown in Figure 9d. The first end 14 is located at the distal-most end of the shaft 70. The second end 16 is located proximally on the shaft 70.

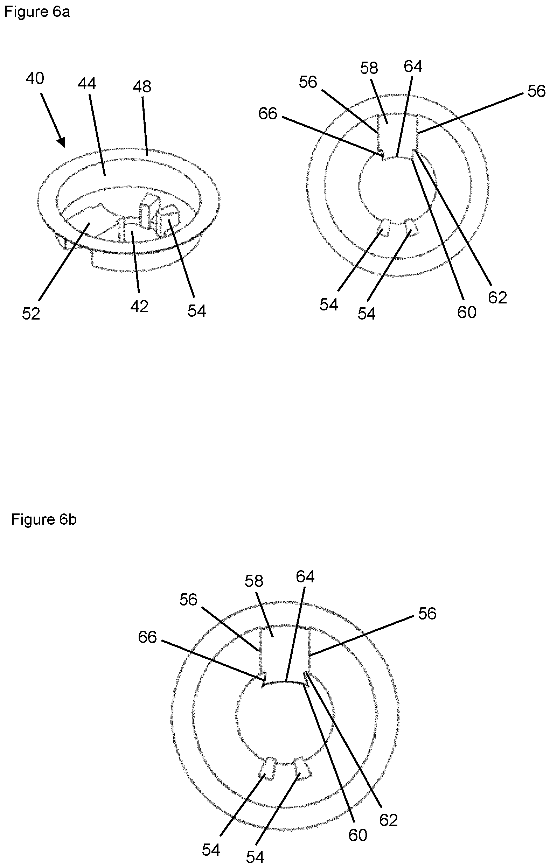

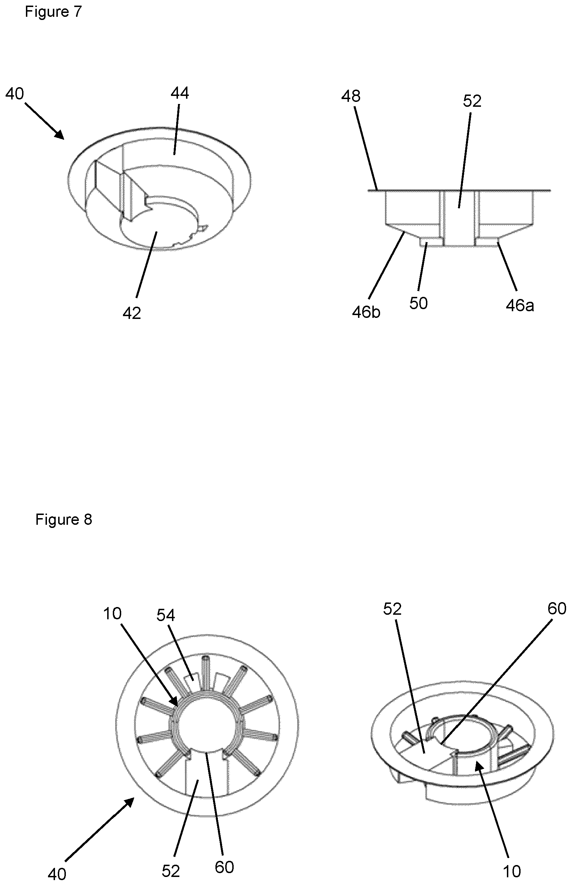

The accessory 10 may be provided in a holder which facilitates fitting the accessory to a shaft 70 of a medical device. As shown in Figures 6a, 6b and 7, the holder may comprise a container 40 in which an accessory 10 may be stored prior to use. The container 40 may be generally cylindrical and comprises a flat circular base 42 and a perimeter wall 44, together defining an enclosure in which the cover can be located. An annular lip 48 may extend around the upper edge of the perimeter wall 44. In use, a peel-off cover (not shown) for the container 40 may be adhered to the lip 48.

One or more additional walls may be provided between the base 42 and the perimeter wall 44. In this example, a short vertical wall 46a is provided around the flat base wall 42 and a sloping wall 46b is provided between the vertical wall 46a and the perimeter wall 44. The base 42 and the short vertical wall 46a together define a shallow recess 50 in the base of the container 40 for receiving the first end 14 of the accessory 10 in use. The sloping wall 46b supports the projecting elements 24 of the accessory 10 in use.

The container 40 is further provided with features to locate the accessory 10 in the container 40 in its second, open position. In particular, a blocking element 52 is provided, which projects into the enclosure and at least part of which fits within the axial slot 26 of the tubular body 12 when it is in its second, open position. This prevents the accessory 10 springing back into its closed position under the action of the spring member 28. At least one locating member 54 may be arranged opposite to the blocking element 52, to help correctly position the accessory 10 against the blocking element 52. The (or each) locating member 54 may also be referred to as a locating element 54.

The blocking element 52 comprises a main body in the form of a generally rectangular column projecting radially into the container 40 from the perimeter wall 44 and upwardly from the base of the container 40 (in this example including the base 42, and additional walls 46a, 46b). The main body of the blocking element 52 comprises a pair of side walls 56 joined by an upper surface 58. On its radially inner side, the blocking element 52 is formed with a narrowed nose portion 60 formed by side walls 66 which are joined to the side walls 56 of the main body by a shoulder 62. The end face 64 of the nose portion 60, which joins the side walls 66 and faces radially inwardly, has a concave arcuate form.

As shown in Figure 6a, the side walls 66 of the nose portion 60 may be parallel to each other and to the side walls 56 of the main body of the blocking element 52. Alternatively, in a variant of the container 40 as shown in Figure 6b, the side walls 66 of the nose portion 60 may be non-parallel and may diverge away from each other in a radially inward direction from the shoulder 62 towards the end face 64. This may help to retain the nose portion 60 more securely within the slot 26 of the accessory 10 when the accessory 10 is fitted in the container 40 as discussed further below.

On the opposite side of the container 40, in this example a pair of locating members 54 is provided. Each locating member 54 comprises a raised projection protruding upwardly from the sloping wall 44b and base 42 and extending a small distance into the recess 50.

The blocking element 52 projects upwardly to a first height, which is typically about the same as the axial length of an accessory 10 to be housed in the container 40. The locating member(s) 54 project upwardly to a second height, which is less than the first height of the blocking element 52, typically about half of the first height.

In use, an accessory 10 is deflected into its open position and fitted into the container 40. The tubular body 12 is fitted between the blocking element 52 and the locating members 54. The first end 14 of the tubular body 12 is received in the recess 50. The projecting elements 24 are supported on the sloping wall 46b. The nose portion 60 of the blocking element 52 is located in the slot 26. The curved end face 64 of the nose portion 62 follows the curvature of the inner circumferential surface 20 of the tubular body 12 in order to form a smooth circular opening into which the shaft 70 may be inserted. The locating members 54 fit either side of one of the projecting elements 24. Depending on the configuration of the projecting elements 24, the number, shape and position of the locating members 54 may be altered to suit. In this way, the accessory 10 is held securely in the container 40 in its open position, until it is required for use.

The accessory 10 may be stored in a sterile condition in the container 40 with a cover (not shown) applied across the top of the container 40 and adhered to the lip 48 to seal the container 40. When the accessory 10 is required for use, the cover is removed. As shown in Figures 9a and 9b, the distal end of a shaft 70 of a medical device is inserted into the tubular body 12. Since the tubular body 12 is being held in the open position, the inside diameter of the accessory 10 is larger than the outside diameter of the shaft 70 and the shaft 70 can be easily inserted.

The shaft 70 may then be tilted to one side as shown in Figure 9c, away from the blocking element 52 and towards the locating members 54, in order to remove the nose portion 60 of the blocking element 52 from the slot 26. Since the height of the locating members 54 is less than the height of the blocking element 52, this tilting movement is possible. Once the nose portion 60 is removed from the axial slot 26, the spring member 28 urges the tubular body 12 back into its first, closed position with a reduced internal diameter. In this state, the accessory 10 grips the shaft 70 and together they can be removed from the container 40, leaving the accessory 10 in position on the shaft 70 and ready for use as shown in Figure 9d. The container 40 can then be disposed of.

When the accessory 10 is fitted on a shaft 70, the medical device may then be inserted into a patient's body. Normally when examining the colon, for example, the shaft 70 of the medical device is advanced relatively quickly to the furthest point requiring examination and then gradually withdrawn more slowly, with most of the visual examination carried out during the withdrawal. As the shaft 70 is gradually withdrawn, the projecting elements 24 act to gently pull back and flatten folds of tissue, to allow a clearer view of the body tissues through the device.

At the end of the procedure the shaft 70 is completely withdrawn from the patient's body. The accessory 10 can then be removed from the shaft 70, for example by manually bending the tubular body 12 to open the axial slot 26 so that the accessory 10 can slide off the shaft 70. The accessory 10 can then be disposed of.

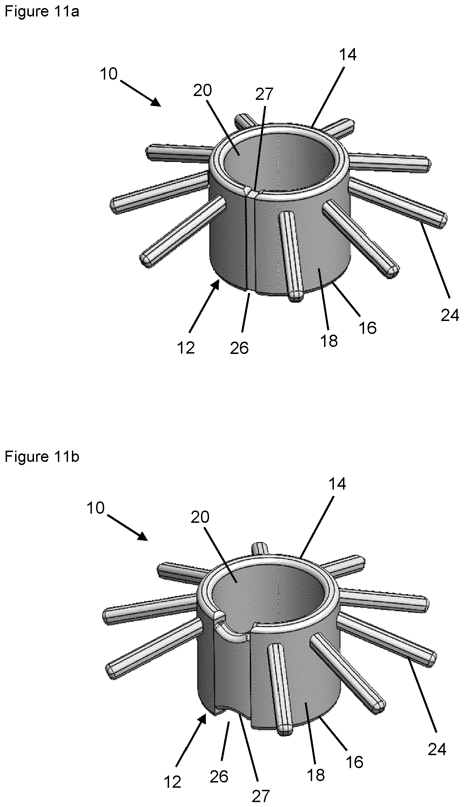

In the accessories 10 as shown in Figures 1 to 10i, the slot 26 is open and axially aligned. Figures 11a to 21b show further examples of accessories 10 with alternative designs. Each accessory 10 may be as discussed herein, including any of the modifications discussed, unless otherwise expressly stated to the contrary.

Figures 11a and 11b show a further example of an accessory 10. With this accessory 10, a bridging element 27 is provided to span at least a portion of the slot 26. Specifically, the bridging element 27 may span the slot 26 in a circumferential direction. This may be between opposite edges of the tubular body 12 which define the slot 26. The tubular body 12, and particularly (where present) the spring member 28, do not extend into this slot 26. Thus, a slot 26 may still be defined.

The bridging element 27 can extend along substantially an entire length of the slot 26, such as shown in Figures 11a and 11b. This may be at least 80%, 90% or even 95% of the length of the slot 26 - or 100% of the length of the slot 26.

The bridging element 27 may be made of an elastic and/or flexible material. That is, specifically a material which is more elastic and/or flexible than the tubular body 12 and/or the spring member 28. The bridging element 27 may be resiliently deformable. This may be with a bias towards the first, closed position. In this sense, it is also possible for the bridging element 27 to be identified as the biasing member 28 - that is, there may be no separate biasing member 28 and the bridging element 27 may be the biasing member 28.

When the tubular body 12 is in the first, closed position as shown in Figure 11a the bridging element 27 spans the slot 26 in the circumferential direction. The bridging element 27 may be substantially un-stressed in this position. The tubular body 12 may then be opened to the second, open position. This opens up the axial slots 26 and stretches the bridging element 27.

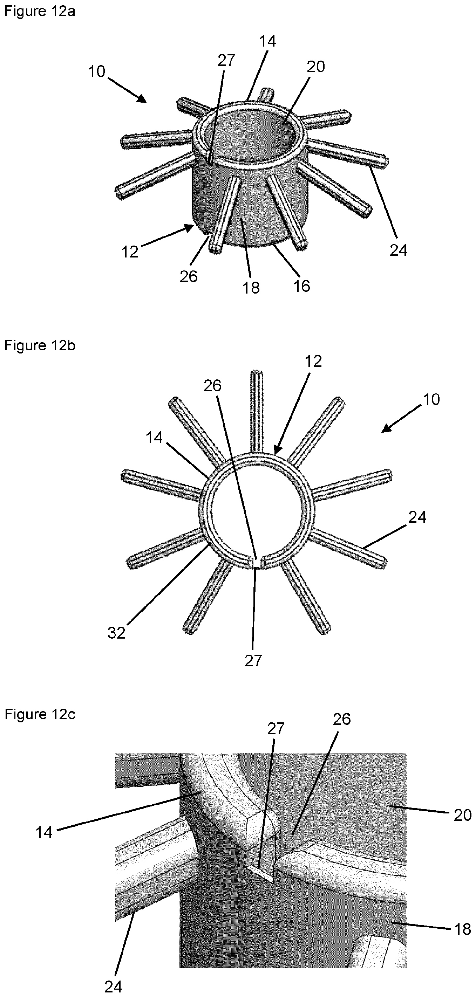

Figures 12a to 12c show that the bridging element 27 may be formed as a sheet. For example, this could be formed as a sheet of the coating material 32 discussed above (with or without a spring member 28). In such an example, the spring member 28 may stop before the slot 26, with the coating material 32 continuing to form the bridging element 27.

Of course, this sheet could be formed of a separate material attached to the tubular body 12. In general, the sheet may be formed of any flexible material.

The sheet forming the bridging element 27 may be thinner than the tubular body 12. For example, the bridging element 27 may have a thickness of less than 25% of a thickness of the tubular body 12, such as less than 15% or less than 10%.

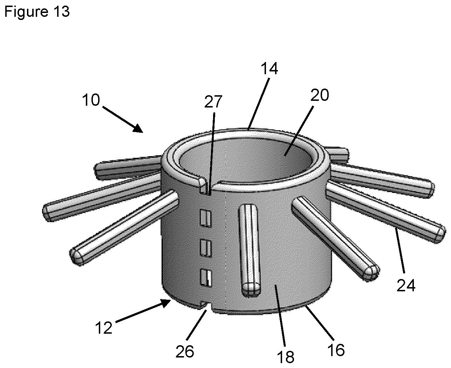

Figure 13 shows a further example accessory 10, with a sheet bridging element 27. This sheet bridging element includes one or more perforations. This means that in the axial direction the bridging element 27 may be discontinuous. In other words, the bridging element 27 is formed of a plurality of bridge sections.

Such an accessory 10 may be formed by only forming the bridge sections in a manufacturing step. Alternatively, a continuous bridging element 27 may be formed, with the perforations then cut therefrom.

While Figure 13 shows this in relation to a thin sheet bridging element 27, this is equally applicable to any of the bridging elements 27 discussed herein.

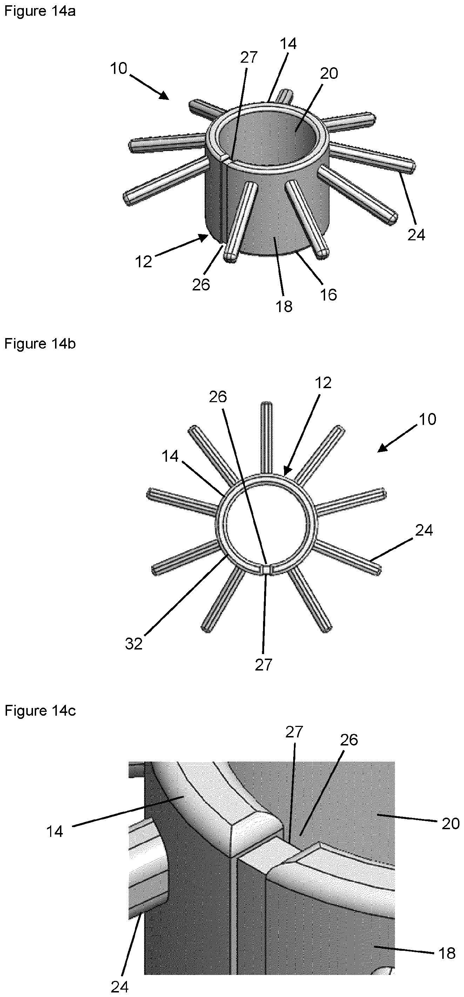

Figures 14a to 14c show a further example accessory 10 where the bridging element 27 has a thickness in the radial direction. This thickness may be 50% or greater of a thickness of the tubular body 12, such as 75% or greater or 85% or greater. In other words, the bridging element 27 substantially fills the slot 26 in the radial direction.

Again, this bridging element 27 may be formed as the same material as a coating material 32, or from a separate material attached to the tubular body 12.

In Figures 14a to 14c (and Figures 11a to 13c) the bridging element 27 substantially extends along a majority of the length of the slot 26. Specifically, this may be an axial length of the slot 26. For example, this could be at least 75% of the length of the slot 26, or at least 85%, or at least 90%. In examples with a discontinuous bridging element 27 this can be defined based on a sum of the lengths of the bridging sections (as a cumulative length), or as a measurement from the outermost end of the bridging section nearest the first end 14 to the outermost end of the bridging section nearest the second end 18.

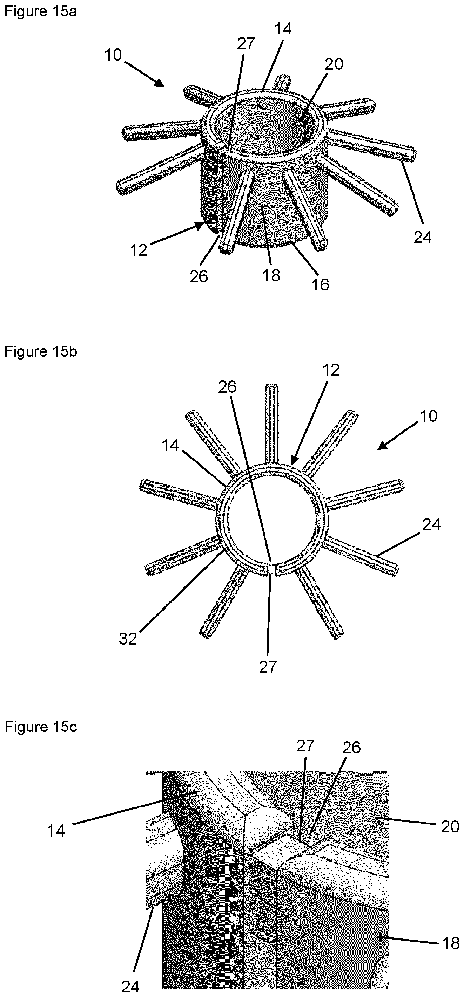

Figures 15a to 15c show an alternative arrangement where the bridging element 27 only spans a portion of the length of the slot 26. This portion may be less than 50% of the length of the slot 26, such as less than 25%, or less than 10%. Otherwise, the bridging element 27 may be as discussed herein in relation to any of the accessories 10. Figures 15a to 15c show a thick bridging element such as in Figures 14a to 14c, but this could equally be applied to any of the bridging elements discussed herein.

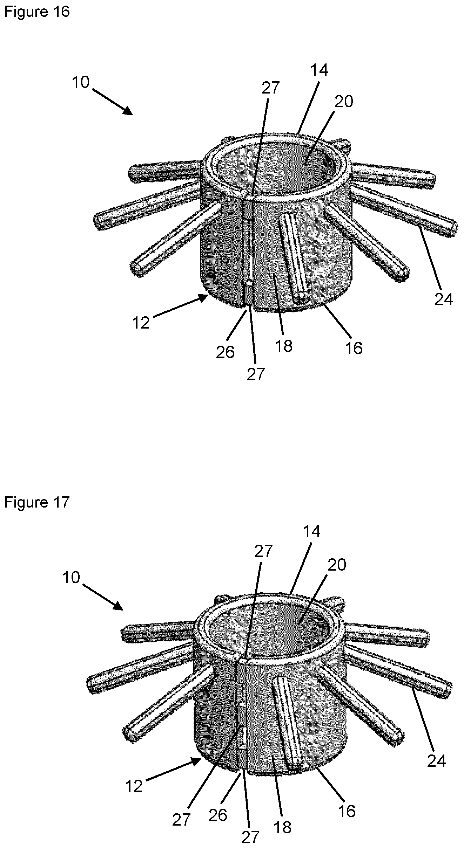

Figures 16 and 17 show an example of accessories 10 with discontinuous bridging elements 27 which is thick such as the bridging element of Figures 14a to 14c. This bridging element 27 comprises a plurality of bridging sections, separated by perforations or gaps. Each bridging section spaced along a length of the slot 26.

Such discontinuous bridging elements 27 may be formed as discussed above in relation to Figure 13.

Any of the disclosures herein relating to the various bridging elements 27 may be used with any of the accessories 10 disclosed unless expressly stated to the contrary.

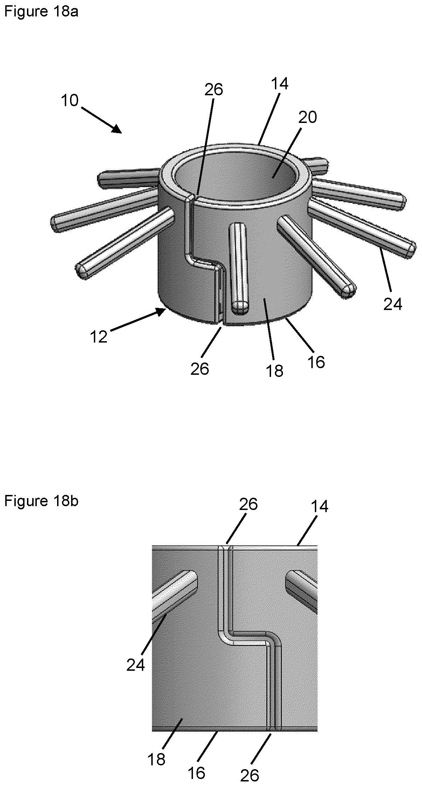

Figures 18a to 21b show further examples of accessories 10 where a shape of the slot 26 has been varied. Unless expressly stated otherwise, any of the bridging elements 27 discussed herein may be used with these different slots 26.

Figures 18a and 18b show an example of an accessory 10 with a non-linear slot 26. This can also be defined as a serpentine slot 26, a tortuous slot 26, an offset slot 26, or a hybrid slot 26. Critically, such a non-linear slot 26 includes at least a portion which is not solely in the axial direction.

For example the accessory 10 of Figures 18a and 18b comprises a first axial portion and a second axial portion, with a connecting circumferential portion. Thus, the first axial portion and the second axial portion are circumferentially offset from one another.

Again, the or each axial portion needs not be purely axial but may also extend in the circumferential direction. An axial portion of the slot 26 may be defined as one that extends further in the axial direction than it does in the circumferential direction.

Likewise, the or each circumferential portion does not need to be purely circumferential, but may also extend in the axial direction. A circumferential portion of the slot 26 extends further in the circumferential direction than it does in the axial direction. However, it is appreciated that this is not necessarily the case and examples may be provided where the circumferential portion still extends more in the axial direction.

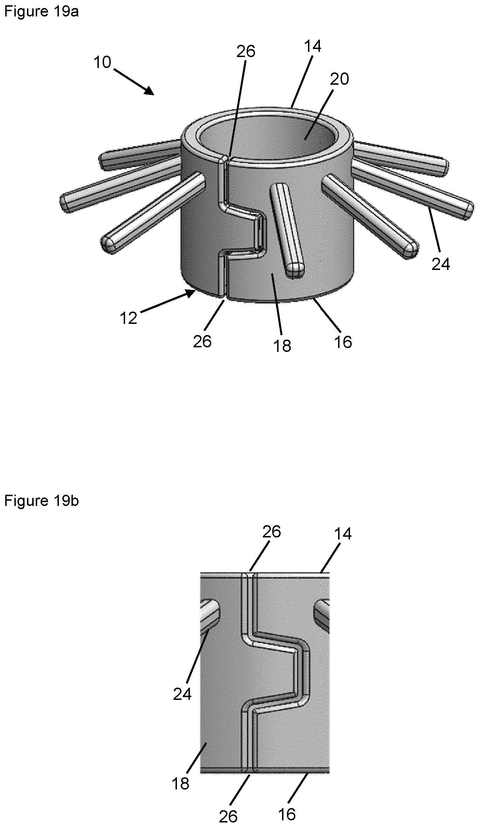

Figures 19a to 19b show a further example of a non-linear slot 26. This slot 26 includes a first axial portion at a first end 14 of the tubular body 12, and a second axial portion at a second end of the tubular body 12. In this example, the first axial portion and the section axial portion are circumferentially aligned, but this is not necessarily the case. The first axial portion and the second axial portion may be the same length as one another, or different lengths.

A third axial portion is provided between the first axial portion and the second axial portion. The third axial portion is circumferentially offset from the first axial portion and the second axial portion. The third axial portion may be the same length as one or both of the first axial portion and/or the second axial portion, or different lengths to one of both of these.

A first circumferential portion extends between the first axial portion and the third axial portion. A second circumferential portion extends between the second axial portion and the third axial portion. The first circumferential portion and the second circumferential portion may be the same length as one another, or different lengths.

The non-linear slot 26 may be substantially symmetric about a mid-section of the tubular body 12.

Such a non-linear slot 26 can incorporate any of the bridging elements 27, or indeed any of the other modifications, discussed herein.

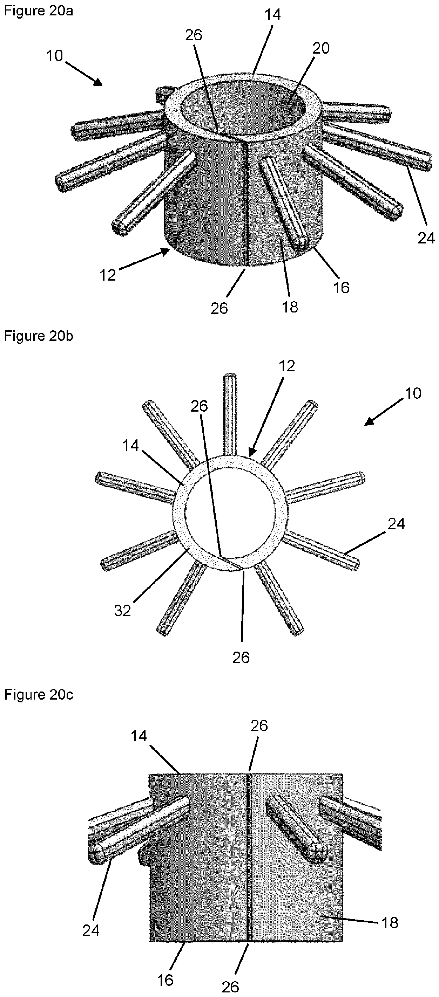

Figures 20a to 20c show an example of an angled slot 26. Specifically, the slot 26 may be angled with respect to a radial direction. This may be at a first angle of at least 20°, preferably at least 30° or at least 45°.

In other words, the slot extends between an inner opening on the inner circumferential surface 20 and an outer opening on the outer circumferential surface 18. The inner opening and the outer opening being circumferentially offset from one another.

Such an angled slot 26 can incorporate any of the bridging elements 27, or indeed any of the other modifications, discussed herein.

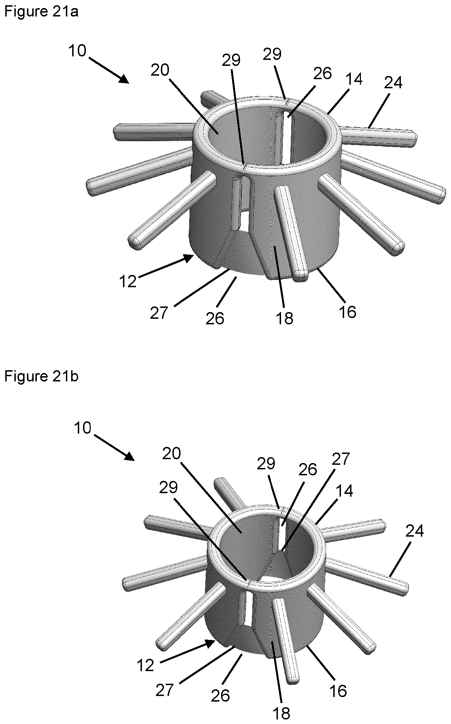

Figures 21a and 21b show an example of a slot 26 which has a varying width. Specifically, the slot 26 is wider towards the second end 16 of the tubular body 12 than at the first end 14 of the tubular body.

In this example, the slot 26 includes an axial portion, which then expands out into a flared portion. The flared portion increasing in width in the direction of the second end 16 of the tubular body 12. Of course, examples with only a flared portion are also possible. Equally, the axial portion may itself increase in width in this direction - potentially at a lesser rate than the flared portion.

A bridging element 27 may be provided. This can span the flared portion of the slot 26, and/or the axial portion (and any section thereof). This bridging element 27 can be as discussed in relation to any of the examples herein.

As can be seen, in this example accessory 10 there is a first slot 26 and a second slot 26. The first slot 26 and the second slot 26 being circumferentially opposite one another about the tubular body 12. Such an arrangement of two slots 26 is equally possible with any of the slots 26 discussed herein. Indeed, arrangements with more than two slots 26 are also possible. In an example with N slots 26, the slots 26 may be arranged with N-fold rotational symmetry about the tubular body 12.

In the example accessory 10 of Figures 21a and 21b, each slot 26 does not extend along a full axial extent of the tubular body 12. For example, this could be at least 75% of the axial length of the tubular body 12, at least 80%, or at least 90%. In this sense, the slot 26 can be identified as a partial slot. Such a partial slot can be used with any of the accessories 10 discussed herein.

Aligned circumferentially with each slot 26 is a hinge section 29. This hinge section 29 arranged at a first end 14 of the tubular body 12. This may be, for example a living hinge. The hinge section 29 allows the slot 26 to be opened, by hinging the two parts of the tubular body 12 about the hinge section 29. As shown in Figures 21a and 21b, the hinge section 29 may include a radial slot cut into the tubular body 12.

Thus, the present disclosure provides an improved accessory 10 for the shaft 70 of a medical device. The accessory 10 can be securely stored in a container 40 ready for fitting to a shaft 70 and once in position, grips the shaft 70 securely.

As noted above, in this disclosure the term "medical device" may refer to an endoscope, but this term is also intended to refer to any device suitable for insertion into a biological cavity or lumen in order to carry out visualisation thereof and/or treatment. Accordingly, the term "medical device" is intended to encompass any or all of endoscopes, gastroscopes, colonoscopes, enteroscopes, sigmoidoscopes, panendoscopes, but this list is non-exhaustive. Endoscopy involves inspecting the inside of a body lumen or cavity and includes procedures known as arthroscopy, cystoscopy, gastroscopy, uteroscopy and colonoscopy. Enteroscopy involves examination of the small intestine including the duodenum, jejunum and ileum. Such devices are elongate flexible probes which may be inserted into the body directly or via a cannula or other guide device. Medical devices can also include rigid surgical endoscopes or endotherapy devices such as biopsy forceps and polypectomy snares. The cover of the present disclosure may be used in conjunction with all of the aforementioned types of medical device.

### CLAUSES:

1. A cover for fitting over a distal end of a shaft of a medical device, the cover comprising:
   a tubular body with a first end, a second end, inner and outer circumferential surfaces, an internal diameter and at least one projecting element extending outwardly; and
   an axial slot in the tubular body extending at least partially between the first end and the second end, wherein the tubular body is movable between a first position in which the slot has a first width and the tubular body has a first internal diameter, and a second position in which the width of the slot and the internal diameter of the tubular body are increased, wherein the tubular body is resiliently biased from the second position towards the first position.
2. The cover of clause 1, wherein the axial slot extends a full length of the tubular body between the first end and the second end.
3. The cover of clause 1, wherein the axial slot is a partial axial slot.
4. The cover of clause 3, wherein the tubular body comprises a hinge section aligned circumferentially with the partial axial slot.
5. The cover of any preceding clause, comprising a plurality of axial slots.
6. The cover of clause 5, wherein the axial slots are arranged with rotational symmetry about the tubular body.
7. The cover of any preceding clause, wherein the tubular body further comprises at least one biasing member.
8. The cover of clause 7, wherein the biasing member comprises a resilient spring plate located between the inner and outer circumferential surfaces of the tubular body.
9. The cover of clause 8, wherein the spring plate is perforated.
10. The cover of clause 8 or clause 9, wherein the spring plate comprise a plurality of circumferentially extending bands joined by at least one axially extending connecting bar.
11. The cover of any of clauses 7 to 9, wherein the biasing member comprises a plurality of separate circumferentially extending bands.
12. The cover of any of clauses 7 to 11, wherein the biasing member is formed of metal.
13. The cover of any of clauses 7 to 12, wherein the biasing member is embedded within a flexible coating material.
14. The cover of any of clauses 7 to 13, wherein the biasing member is moulded in one piece with a flexible coating material.
15. The cover of any preceding clause, wherein the axial slot is non-linear.
16. The cover of any preceding clause, wherein the axial slot is angled with respect to a radial direction of the tubular body.
17. The cover of any preceding clause, wherein the axial slot increases in width towards the second end of the tubular body.
18. The cover of any preceding clause, further comprising a flexible bridging element spanning at least a portion of the axial slot.
19. The cover of clause 18, wherein the flexible bridging element is axially discontinuous.
20. A holder for the cover of any preceding clauses, wherein the holder comprises a base and a side wall together defining an enclosure for receiving the cover, and further comprising a blocking member projecting into the enclosure and configured to fit within the axial slot in the tubular body when the tubular body is in the second position, to maintain the tubular body in the second position.
21. The holder of clause 20, wherein the blocking member comprises a main body with a nose portion, wherein the nose portion is narrower than the main body and is configured to fit into the axial slot in the tubular body.
22. The holder of clause 20 or clause 21, further comprising at least one locating member projecting into the enclosure opposite to the blocking member.
23. The holder of clause 22, wherein the blocking member extends upwardly from the base to a first height, the or each locating member extends upwardly from the base to a second height, and the first height is greater than the second height.
24. The holder of any of clauses 20 to 23, wherein the nose portion comprises parallel side walls.
25. The holder of any of clauses 20 to 23, wherein the nose portion comprises diverging side walls.
26. The holder of any of clauses 20 to 25, wherein the nose portion comprises a concave end face.
27. An assembly comprising the cover of any of clauses 1 to 19 fitted into the holder of any of clauses 20 to 26.
28. A method for fitting the cover of any of clauses 1 to 19 to the distal end of a shaft of a medical device, wherein the cover is fitted into the holder of any of clauses 20 to 26 such that the blocking member locates in the axial slot of the tubular body to maintain the cover in the second position, and the method comprises inserting the distal end of a shaft of a medical device into the tubular body, tilting the shaft to remove the blocking member from the axial slot and to permit the tubular body to return to the first position and to grip the shaft, and removing the shaft and cover from the holder.

## Claims

1. A cover for fitting over a distal end of a shaft of a medical device, the cover comprising:
a tubular body with a first end, a second end, inner and outer circumferential surfaces, an internal diameter and at least one projecting element extending outwardly; and
an axial slot in the tubular body extending at least partially between the first end and the second end, wherein the tubular body is movable between a first position in which the slot has a first width and the tubular body has a first internal diameter, and a second position in which the width of the slot and the internal diameter of the tubular body are increased, wherein the tubular body is resiliently biased from the second position towards the first position.

2. The cover of claim 1, wherein the axial slot:
extends a full length of the tubular body between the first end and the second end; or
is a partial axial slot, preferably the tubular body comprises a hinge section aligned circumferentially with the partial axial slot.

3. The cover of any preceding claim, comprising a plurality of axial slots, preferably the axial slots are arranged with rotational symmetry about the tubular body.

4. The cover of any preceding claim, wherein the tubular body further comprises at least one biasing member,
preferably the biasing member comprises a resilient spring plate located between the inner and outer circumferential surfaces of the tubular body, the spring plate: optionally being perforated; and/or optionally comprising a plurality of circumferentially extending bands joined by at least one axially extending connecting bar.

5. The cover of claim 4, wherein the biasing member comprises a plurality of separate circumferentially extending bands.

6. The cover of any of claims 4 to 5, wherein the biasing member is formed of metal.

7. The cover of any of claims 4 to 6, wherein the biasing member:
is embedded within a flexible coating material; and/or
is moulded in one piece with a flexible coating material.

8. The cover of any preceding claim, wherein the axial slot:
is non-linear; and/or
is angled with respect to a radial direction of the tubular body; and/or increases in width towards the second end of the tubular body.

9. The cover of any preceding claim, further comprising a flexible bridging element spanning at least a portion of the axial slot,
preferably the flexible bridging element is axially discontinuous.

10. A holder for the cover of any preceding claim, wherein the holder comprises a base and a side wall together defining an enclosure for receiving the cover, and further comprising a blocking member projecting into the enclosure and configured to fit within the axial slot in the tubular body when the tubular body is in the second position, to maintain the tubular body in the second position,
preferably the blocking member comprises a main body with a nose portion, wherein the nose portion is narrower than the main body and is configured to fit into the axial slot in the tubular body.

11. The holder of claim 10, further comprising at least one locating member projecting into the enclosure opposite to the blocking member,
preferably the blocking member extends upwardly from the base to a first height, the or each locating member extends upwardly from the base to a second height, and the first height is greater than the second height.

12. The holder of any of claims 10 to 11, wherein the nose portion comprises:
parallel side walls; or
diverging side walls.

13. The holder of any of claims 10 to 12, wherein the nose portion comprises a concave end face.

14. An assembly comprising the cover of any of claims 1 to 9 fitted into the holder of any of claims 10 to 13.

15. A method for fitting the cover of any of claims 1 to 9 to the distal end of a shaft of a medical device, wherein the cover is fitted into the holder of any of claims 10 to 13 such that the blocking member locates in the axial slot of the tubular body to maintain the cover in the second position, and the method comprises inserting the distal end of a shaft of a medical device into the tubular body, tilting the shaft to remove the blocking member from the axial slot and to permit the tubular body to return to the first position and to grip the shaft, and removing the shaft and cover from the holder.
